Europäisches Patentamt

European Patent Office

Office européen des brevets

(11) Numéro de publication : **0 319 407 B1**

# (12) FASCICULE DE BREVET EUROPEEN

(45) Date de publication du fascicule du brevet :
06.05.92 Bulletin 92/19

(51) Int. Cl.⁵ : **C07C 403/08**

(21) Numéro de dépôt : **88403017.2**

(22) Date de dépôt : **30.11.88**

(54) **Procédé de préparation de la vitamine A.**

(30) Priorité : **01.12.87 FR 8716629**

(43) Date de publication de la demande :
07.06.89 Bulletin 89/23

(45) Mention de la délivrance du brevet :
06.05.92 Bulletin 92/19

(84) Etats contractants désignés :
**AT BE CH DE ES FR GB GR IT LI LU NL SE**

(56) Documents cités :
FR-A- 1 169 570
SU-A- 1 141 170
US-A- 2 839 585

(56) Documents cités :
**JOURNAL OF ORGANOMETALLIC CHEMIS-
TRY, vol. 193, 1980, pages C63-C66, Elsevier
Sequoia S.A., Lausanne, CH; W. STROHMEIER
etal.: "Selektive Bulkhydrierung von alpha-
beta-ungesättigten Aldehyden zu ungesättigten Alkoholen mit homogenenRutheniumkatalysatoren"**

(73) Titulaire : **RHONE-POULENC SANTE
20, avenue Raymond Aron
F-92160 Antony (FR)**

(72) Inventeur : **Grosselin, Jean-Michel
15 rue Terraille
F-69001 Lyon (FR)**
Inventeur : **Mercier, Claude
85 avenue du Point du Jour
F-69005 Lyon (FR)**

(74) Mandataire : **Le Pennec, Magali et al
RHONE-POULENC SANTE, Service Brevets,
20 Avenue Raymond Aron
F-92165 Antony Cédex (FR)**

## Description

La présente invention concerne un procédé de préparation de la vitamine A par réduction du rétinal.

La vitamine A [diméthyl-3,7 (triméthyl-2,6,6 cyclohexène-1 yl)-9 nonatétraène-2,4,6,8 ol-1], ainsi que l'aldéhyde de la vitamine A (ou rétinène), présentent le maximum d'activité biologique lorsqu'ils sont sous forme 2,6-trans,trans.

L'aldéhyde de la vitamine A sous forme 2,6-trans,trans peut être séparée des autres isomères par formation d'un complexe avec l'hydroquinone (brevet américain US 2 683 746).

La vitamine A peut être obtenue par réduction de l'aldéhyde de la vitamine A éventuellement sous forme de complexe avec l'hydroquinone. Cependant la réduction de la fonction aldéhyde est souvent accompagnée d'une isomérisation des doubles liaisons en 2 et 6.

D'après le brevet américain US 2 839 585 et d'après T. Mukaiyama et A. Ishida, Chem. Letters, 1975, p. 1201, le complexe de l'aldéhyde de la vitamine A tout-trans avec l'hydroquinone est réduit en vitamine A tout-trans, avec de bons rendements, au moyen de borohydrure de sodium dans le méthanol.

Il est connu de préparer la vitamine A par hydrogénation catalytique du rétinène.

D'après le brevet français FR 2 234 258, la réduction peut être effectuée en présence d'un catalyseur à base de platine et de cobalt en opérant dans un alcool aliphatique. Cependant ce procédé, qui nécessite la mise en oeuvre d'une quantité importante de platine, conduit à une vitamine A dans laquelle le rapport 2-cis/tout-trans est voisin de 1/7.

Dans le brevet russe SU 1 141 170, il est proposé d'effectuer la réduction en présence d'un catalyseur complexe $Ir/Co/BaO/\gamma\text{-}Al_2O_3$. Cependant, si ce procédé conduit à de très bons rendements avec une stéréoisomérie tout-trans unique, il nécessite l'emploi d'un catalyseur à base d'iridium qui est onéreux et peu productif.

D'après G. Mestroni et coll., "Homogeneous Catalytic Reduction of Carbonyl-, Azomethine -and Nitro - Groups" in "Aspect of Homogeneous Catalysis" par R. Ugo, 1981, vol. 4, p. 74, édité par D. Reidel (Hollande), il est connu d'hydrogéner les aldéhydes linéaires ou ramifiés ou aromatiques en présence de complexe $RuCl_2$ $(CO)_2$ $[P(C_6H_5)_3]_2$ dans des conditions qui, essentiellement dans le cas des aldéhydes linéaires, conduisent fréquemment à des réactions secondaires. D'après W. Strohmeier et K. Holke, J. Organometal. Chem., 193, c63-c66 (1980) l'hydrogénation sélective des aldéhydes $\alpha,\beta$-insaturés en alcools insaturés est, de préférence, effectuée en utilisant le complexe du ruthénium $RuCl_2$ $(CO)_2$ $[P(C_6H_{11})_3]_2$ les hydrures de ruthénium $[RuH_2$ $[P(C_6H_5)_3]_4]$ ne conduisant pas à des résultats satisfaisants.

Il a maintenant été trouvé, et c'est ce qui fait l'objet de la présente invention, que l'hydrogénation du rétinal tout-trans, éventuellement sous forme de complexe avec l'hydroquinone, en rétinol tout-trans peut être réalisée sélectivement, sans réaction secondaire d'isomérisation, en utilisant un catalyseur choisi parmi les hydrures de ruthénium éventuellement générés in situ, éventuellement en présence d'une base ou d'un milieu tampon nécessaire pour neutraliser l'acidité du milieu.

Les hydrures de ruthénium sont associés à un ligand (L) qui est choisi de préférence parmi les dérivés phosphorés (phosphines, phosphites), oxygénés (acétate, trifluoroacétate), azotés (hydroxypyridines), soufrés ou silylés.

Les catalyseurs utilisables sont la mise en oeuvre du procédé selon l'invention peuvent être des composés bien définis, comme par exemple $RuH_2$ $(PPh_3)_4$, ou être générés in situ à partir de précurseurs tels que Ru $(C_8H_8)_2$ $(PPh_3)_2$ ou $RuCl_2$ $(PPh_3)_4$.

D'un intérêt tout particulier sont les hydrures de ruthénium $RuH_2$ $(PPh_3)_4$, RuH (OAc) $(PPh_3)_4$ ou RuH (hydroxy-2 pyridine) $(PPh_3)_3$.

Le catalyseur $RuH_2$ $(PPh_3)_4$ peut être préparé dans les conditions décrites par R.O. Harris et coll., J. Organometal. Chem., 54, 259-264 (1973) et le catalyseur RuH (OAc) $(PPh_3)_4$ peut être préparé selon Inorg. Synthesis, 16, p. 53 et 75 à 79 ou selon Comprehensive Organometallic Chemistry, Vol. IV, Chapitre 32.3.2 p. 717.

Le catalyseur $RuH_2$ $(PPh_3)_3$ peut être généré in situ par la méthode décrite par G. Wilkinson et coll., J. Chem. Soc, Dalton, 1739 (1978) à partir de $Ru(C_8H_8)_2$ $(PPh_3)_3$ ou par la méthode décrite dans J. Organometal. Chem., 142, C55-57 (1977) à partir de $RuCl_2$ $(PPh_3)_4$.

Généralement, l'hydrogénation est réalisée à une température comprise entre 0 et 100°C, de préférence entre 20 et 50°C, en opérant dans un solvant organique. Il est particulièrement avantageux d'opérer sous une pression comprise entre 1 et 200 bars et, de préférence entre 1 et 20 bars. Généralement la réduction est complète après une heure de réaction.

Comme solvant organique, on utilise de préférence un solvant polaire choisi parmi les alcools (méthanol, éthanol, isopropanol) éventuellement associé à un solvant non polaire choisi parmi les hydrocarbures aliphatiques (pentane, hexane, heptane, octane), alicycliques (cyclohexane), aromatiques (benzène, toluène, xylène), les éthers (éther diéthylique, diisopropylique, tétrahydrofuranne, dioxanne) ou les esters (acétate de méthyle, acétate d'éthyle, acétate de butyle).

Il est particulièrement avantageux d'ajouter au solvant polaire ou au mélange de solvants une quantité d'eau pouvant atteindre jusqu'à 10 % du volume total afin d'éviter en particulier la formation d'acétal.

Généralement, on utilise de 0,1 à 0,0001 mole, de préférence de 0,03 à 0,003 mole de catalyseur par mole d'aldéhyde de vitamine A mise en oeuvre.

Les exemples suivants, donnés à titre non limitatif, montrent comment l'invention peut être mise en pratique.

## EXEMPLE 1

Dans un réacteur homothétique de 250 cm3, on introduit à 25°C, sous atmosphère d'argon, 708 mg (0,61 mmole) de $RuH_2$ $[P(C_6H_5)_3]_4$ puis 2,303 g de complexe de l'aldéhyde de la vitamine A avec l'hydroquinone (complexe RHQ) soit 6,4 mmoles de rétinal. (Le complexe RHQ titre 79,6 % et contient 96,1 % d'isomère tout-trans, 3,5 % d'isomère 2-cis et 0,4 % d'isomère 6-cis ainsi que 1,1 % d'iode et 252 ppm de soufre). On dissout, à l'abri de la lumière, dans 100 cm3 d'isopropanol contenant 2 cm3 d'eau. On purge à l'hydrogène sous une pression de 1 bar pendant 15 minutes. On agite alors le mélange réactionnel à 1000 tours/minute. La réaction est suivie par le volume d'hydrogène absorbé et par chromatographie liquide à haute performance [colonne Si60 Lichrosorb 125 x 4 - Hexane - acétate d'éthyle (90-10 en volumes) - Absorption à 325 nm - Débit : 1 cm-3/minute].

Après 1 heure, le volume d'hydrogène absorbé est de 155 cm3 (théorie : 154 cm3).

La chromatographie liquide à haute performance (CLHP) montre que :
– le taux de transformation du rétinal est supérieur ou égal à 99 %,
– la répartition isomérique du rétinol obtenu est la suivante :
- rétinol tout-trans         : 96,2 %
- rétinol 2-cis              : 3,3 %
- rétinol 6-cis              : 0,5 %
Le rendement est voisin de 94 %.

## EXEMPLE 2

Dans un autoclave de 125 cm3 en hastelloy C, on introduit 67 mg (0,058 mmole) de $RuH_2$ $[P(C_6H_5)_3]_4$, 2,302 g de complexe RHQ. L'autoclave est purgé puis on introduit 50 cm3 d'isopropanol contenant 1 cm3 d'eau. On établit la pression d'hydrogène à 20 bars puis démarre l'agitation à une température voisine de 20°C. On poursuit l'agitation pendant 40 minutes c'est-à-dire jusqu'à la fin de l'absorption d'hydrogène. Après dégazage, on obtient un liquide brun clair homogène dont l'analyse par CLHP montre que :
– le taux de transformation du rétinal est supérieur ou égal à 99,9 %,
– la répartition isomérique du rétinol obtenu est la suivante :
- rétinol tout-trans         : 96,0 %
- rétinol 2-cis              : 3,6 %
- rétinol 6-cis              : 0,4 %
Le rendement est voisin de 94 %.

## EXEMPLE 3 (exemple comparatif)

On opère dans les conditions de l'exemple 13 du brevet français FR 2 234 258 en utilisant un catalyseur $PtO_2/Co(OAc)_2$, $4H_2O$ préréduit et le même complexe RHQ que dans l'exemple 1.

Après 40 minutes de réaction sous une pression de 1 bar à 25°C, l'analyse par CLHP montre que :
– le taux de transformation du rétinal est voisin et 78 %,
– la répartition isomérique du rétinol obtenu est la suivante :
- rétinol tout-trans         : 82 %
- rétinol 2-cis              : 11,8 %
- rétinol 6-cis              : 6,2 %
Le rendement est voisin de 52 %.

## EXEMPLE 4

Dans un autoclave de 125 cm3 en hastelloy C, on introduit 53 mg de RuH (OAc) $[P(C_6H_5)_3]_4$ et 4,6361 g de complexe RHQ. On purge l'autoclave puis on ajoute 50 cm3 de méthanol et 1 cm3 d'eau. On établit la pression d'hydrogène à 20 bars puis agite pendant 3 heures 45 minutes à une température voisine de 20°C. Après dégazage, on obtient un liquide brun clair homogène dont l'analyse par CLHP montre que :
– le taux de transformation du rétinal est voisin de 99,5 %,
– la répartition isomérique du rétinol obtenu est la suivante :
- rétinol tout-trans : 96,2 %
- rétinol 2-cis : 3,3 %
- rétinol 6-cis : 0,5 %
Le rendement est voisin de 92,5 %.

EXEMPLE 5

Dans un autoclave de 125 cm3 en hastelloy C, on introduit 59,3 mg de $RuH_2 [P(C_6H_5)_3]_4$ et 4,6250 g de complexe RHQ. L'autoclave est purgé puis on introduit 50 cm3 de méthanol et 1 cm3 d'eau. On établit la pression d'hydrogène à 20 bars. On place l'autoclave dans le four préchauffé à 50°C puis on agite pendant 20 minutes. Après refroidissement et dégazage, on obtient un liquide brun clair dont l'analyse par CLHP montre que :
– le taux de transformation du rétinal est supérieur à 99,9 %,
– la répartition isomérique du rétinol obtenu est la suivante :
- rétinol tout-trans : 97,4 %
- rétinol 2-cis : 2,1 %
- rétinol 6-cis : 0,5 %
Le rendement est voisin de 96 %.

EXEMPLE 6

Dans un autoclave de 125 cm3 en hastelloy C, on introduit 59,4 mg de $RuH_2 [P(C_6H_5)_3]_3$ et 4,6198 g de complexe RHQ contenant 12,34 mmoles d'aldéhyde de vitamine A tout-trans. L'autoclave est purgé puis on introduit 50 cm3 de méthanol et 1 cm3 d'eau. On établit la pression d'hydrogène à 20 bars à une température voisine de 20°C. On agite pendant 1 heure 10 minutes. Après dégazage, on obtient un liquide brun clair homogène dont l'analyse par CLHP montre que :
– le taux de transformation du rétinal est voisin de 99,9 %,
– la répartition isomérique du rétinol obtenu est la suivante :
- rétinol tout-trans : 95,8 %
- rétinol 2-cis : 3,8 %
- rétinol 6-cis : 0,4 %
Le rendement est voisin de 97 %.

EXEMPLE 7

On opère comme dans l'exemple 6, mais en utilisant 0,893 g de rétinal contenant 95 % d'isomère tout-trans et 21,1 mg de $RuH_2 [P(C_6H_5)_3]_3$ en solution dans 20 cm3 de méthanol et 0,4 cm3 d'eau. On établit la pression d'hydrogène à 50 bars à 50°C. On agite pendant 2 heures 10 minutes. Après dégazage, on obtient un liquide dont l'analyse par CLHP montre que :
– le taux de transformation du rétinal est voisin de 100 %,
– la répartition isomérique du rétinol obtenu est la suivante :
- rétinol tout-trans : 96 %
- rétinol 2-cis : 2,5 %
- rétinol 6-cis : 0,45 %
Le rendement en rétinol tout-trans est voisin de 85 %.

EXEMPLE 8

On opère comme dans l'exemple 5, en utilisant comme solvant de l'éthanol à 95 %. On hydrogène sous une pression de 20 bars à 50°C pendant 20 minutes. Après dégazage, on obtient un liquide dont l'analyse par CLHP montre que :
– le taux de transformation du rétinal est de 98 %,
– la répartition isomérique du rétinol obtenu est la suivante :
- rétinol tout-trans : 95,8 %
- rétinol 2-cis : 3,1 %
- rétinol 6-cis : 0,4 %

Le rendement en rétinol tout-trans est de 92,8 %.

EXEMPLE 9

On opère comme dans l'exemple 5 mais en utilisant 59 mg de RuH (hydroxy-2 pyridine) $(PPh_3)_3$ et 4,62 g de complexe RHQ. L'autoclave est purgé puis on introduit 50 cm3 d'éthanol à 95 %. On établit la pression d'hydrogène à 20 bars à une température voisine de 50°C puis on agite pendant 30 minutes. Après refroidissement et dégazage, on obtient un liquide brun homogène dont l'analyse par CLHP montre que :
– le taux de transformation du rétinal est de 37 %
– le rétinol tout-trans représente 90,5 % du mélange des rétinols
Le rendement en rétinol tout-trans est voisin de 35 %.

EXEMPLE 10 (exemple comparatif)

Dans un autoclave de 125 cm3 en hastelloy C, on charge 72,8 mg de $RuCl_2$ $[P(C_6H_5)_3]_4$ et 2,3050 g de complexe RHQ. On purge l'autoclave puis on ajoute 50 cm3 d'isopropanol et 1 cm3 d'eau. On établit la pression d'hydrogène à 20 bars à une température voisine de 20°C puis on agite pendant 3 heures. Après dégazage, on obtient un liquide brun clair qui devient rapidement rouge foncé. L'analyse par CLHP du liquide obtenu montre que :
– le taux de transformation du rétinal est de 46 %,
– la répartition isomérique du rétinol obtenu est la suivante :
- rétinol toute-trans        : 71,5 %
- rétinol 2-cis              : 28,5 %
Le rendement en rétinol tout-trans est voisin de 5 %.

**Revendications**

1. Procédé de préparation de la vitamine A pratiquement exempte d'isomère 2-cis et 6-cis par réduction de l'aldéhyde de la vitamine A éventuellement sous forme de complexe avec l'hydroquinone au moyen d'hydrogène caractérisé en ce que l'on opère dans un solvant organique polaire en présence d'un catalyseur choisi parmi les hydrures de ruthénium éventuellement générés in situ, éventuellement en présence d'une base ou d'un milieu tampon nécessaire pour neutraliser l'acidité du milieu.

2. Procédé selon la revendication 1 caractérisé en ce que l'hydrure de ruthénium est associé à un ligand choisi parmi les dérivés phosphorés tels que les phosphines et les phosphites, oxygénés tels que les acétates et trifluoroacétates, azotés tels que les hydroxypyridines, soufrés ou silylés.

3. Procédé selon la revendication 1 caractérisé en ce que le solvant organique polaire est choisi parmi les alcools contenant 1 à 3 atomes de carbone éventuellement associé à un solvant organique non polaire choisi parmi les hydrocarbures aliphatiques, alicycliques ou aromatiques, les éthers et les esters.

4. Procédé selon la revendication 3 caractérisé en ce que l'on ajoute une quantité d'eau pouvant atteindre 10 % du volume total du solvant.

5. Procédé selon la revendication 1 caractérisé en ce que l'on opère sous une pression d'hydrogène comprise entre 1 et 200 bars.

6. Procédé selon la revendication 5 caractérisé en ce que l'on opère sous une pression d'hydrogène comprise entre 1 et 20 bars.

7. Procédé selon la revendication 1 caractérisé en ce que l'on opère à une température comprise entre 0 et 100°C.

8. Procédé selon la revendication 7 caractérisé en ce que l'on opère à une température comprise entre 20 et 50°C.

9. Procédé selon la revendication 1 caractérisé en ce que l'on utilise de 0,1 à 0,0001 mole de catalyseur par mole de rétinal.

10. Procédé selon la revendication 9 caractérisé en ce que l'on utilise de 0,03 à 0,003 mole de catalyseur par mole de rétinal.

11. Procédé selon la revendication 1 caractérisé en ce que le catalyseur est choisi parmi $RuH_2$ $(PPh_3)_4$, RuH $(OAc)(PPh_3)_4$ et RuH (hydroxy-2 pyridine)$(PPh_3)_3$.

5

## Claims

1. A process for the preparation of vitamin A containing practically no 2-cis or 6-cis isomer by reduction of the aldehyde of vitamin A, if appropriate in the form of a complex with hydroquinone, by means of hydrogen, which comprises carrying out the reaction in a polar organic solvent in the presence of a catalyst selected from ruthenium hydrides, which may be generated in situ, if necessary in the presence of a base or a buffer medium for neutralising the acidity of the medium.

2. The process according to claim 1, wherein the ruthenium hydride is associated with a ligand selected from phosphorus derivatives such as phosphines and phosphites, oxygen derivatives such as acetates and trifluoroacetates, nitrogen derivatives such as hydroxypyridines, sulphur derivatives and silylated derivatives.

3. The process according to claim 1, wherein the polar organic solvent is selected from alcohols containing 1 to 3 carbon atoms and is optionally associated with a non-polar organic solvent selected from aliphatic, alicyclic and aromatic hydrocarbons, ethers and esters.

4. The process according to claim 3, wherein an amount of water is added which can be as much as 10% of the total volume of the solvent.

5. The process according to claim 1, wherein the reaction is carried out under a hydrogen pressure of between 1 and 200 bar.

6. The process according to claim 5, wherein the reaction is carried out under a hydrogen pressure of between 1 and 20 bar.

7. The process according to claim 1, wherein the reaction is carried out at a temperature of between 0 and 100°C.

8. The process according to claim 7, wherein the reaction is carried out at a temperature of between 20 and 50°C.

9. The process according to claim 1, wherein from 0.1 to 0.0001 mol of catalyst is used per mol of retinal.

10. The process according to claim 9, wherein from 0.03 to 0.003 mol of catalyst is used per mol of retinal.

11. The process according to claim 1, wherein the catalyst is selected from $RuH_2(PPh_3)_4$, $RuH(OAc)(PPh_3)_4$ and $RuH(2\text{-hydroxypyridine})(PPh_3)_3$.

## Patentansprüche

1. Verfahren zur Herstellung von Vitamin A, das praktisch frei von 2-cis- und 6-cis-Isomerem ist, durch Reduktion des Aldehyds von Vitamin A, gegebenenfalls in Form des Komplexes mit Hydrochinon, mittels Wasserstoff, dadurch gekennzeichnet, daß man in einem polaren organischen Lösungsmittel in Gegenwart eines Katalysators, ausgewählt aus den gegebenenfalls in situ gebildeten Rutheniumhydriden, gegebenenfalls in Gegenwart einer Base oder eines zur Neutralisation des Säuregehalts des Milieus notwendigen Puffermilieus, arbeitet.

2. Verfahren nach Anspruch 1, dadurch gekennzeichnet, daß das Rutheniumhydrid mit einem Liganden, ausgewählt aus den phosphorhaltigen Derivaten, z.B. den Phosphinen und den Phosphiten, den sauerstoffhaltigen Derivaten, z.B. den Acetaten und Trifluoracetaten, den stickstoffhaltigen Derivaten, z.B. den Hydroxypyridinen, den schwefelhaltigen oder silylierten Derivaten, assoziiert ist.

3. Verfahren nach Anspruch 1, dadurch gekennzeichnet, daß das polare organische Lösungsmittel ausgewählt ist aus den Alkoholen mit 1 bis 3 Kohlenstoffatomen, gegebenenfalls kombiniert mit einem nicht polaren organischen Lösungsmittel, ausgewählt aus den aliphatischen, alicyclischen oder aromatischen Kohlenwasserstoffen, den Äthern und den Estern.

4. Verfahren nach Anspruch 3, dadurch gekennzeichnet, daß man eine Wassermenge zusetzt, die 10 % des Gesamtvolumens des Lösungsmittels erreichen kann.

5. Verfahren nach Anspuch 1, dadurch gekennzeichnet, daß man unter einem Wasserstoffdruck zwischen 1 und 200 bar arbeitet.

6. Verfahren nach Anspruch 5, dadurch gekennzeichnet, daß man unter einem Wasserstoffdruck zwischen 1 und 20 bar arbeitet.

7. Verfahren nach Anspruch 1, dadurch gekennzeichnet, daß man bei einer Temperatur zwischen 0 und 100°C arbeitet.

8. Verfahren nach Anspruch 7, dadurch gekennzeichnet, daß man bei einer Temperatur zwischen 20 und 50°C arbeitet.

9. Verfahren nach Anspuch 1, dadurch gekennzeichnet, daß man 0,1 bis 0,0001 mol Katalysator pro mol Retinal verwendet.

10. Verfahren nach Anspruch 9, dadurch gekennzeichnet, daß man 0,03 bis 0,003 mol Katalysator pro Mol Retinal verwendet.

11. Verfahren nach Anspruch 1, dadurch gekennzeichnet, daß der Katalysator ausgewählt ist aus $RuH_2$ $(PPh_3)_4$, RuH (OAc)$(PPh_3)_4$ und RuH (2-Hydroxypyridin) $(PPh_3)_3$.